## Europäisches Patentamt
## European Patent Office
## Office européen des brevets

(11) Publication number: **0 006 281**
A1

# EUROPEAN PATENT APPLICATION

(21) Application number: 79300565.3

(51) Int. Cl.³: **A 61 N 1/36**

(22) Date of filing: 05.04.79

(30) Priority: 07.04.78 US 894358
31.07.78 US 929315

(43) Date of publication of application: 09.01.80
Bulletin 80/1

(84) Designated Contracting States: BE GB IT NL SE

(71) Applicant: MEDTRONIC, INC., 3055 Old Highway Eight
P.O. Box 1453, Minneapolis Minnesota 55440 (US)

(72) Inventor: Coury, Arthur J., 2225 Hillside Avenue, St.
Paul Minnesota 55108 (US)
Inventor: Wilary, Frank J., 11900 54th Avenue North,
Plymouth Minnesota 55442 (US)
Inventor: Ware, Lyle A., 3617 West 102 Street,
Bloomington Minnesota 55431 (US)

(74) Representative: Boyes, Kenneth Aubrey et al, Frank B.
Dehn & Co. Imperial House 15-19 Kingsway, London
WC2B 6UZ (GB)

(54) Body implantable stimulator and connector therefor.

(57) A body implantable stimulator, e.g. a heart pacemaker comprises a signal generator (10), a preformed electrical connector (12) and at least one electrical lead (11) electrically and mechanically connected to the signal generator by preformed electrical connector (12). The connector carries at least one terminal (40) to establish electrical contact between the signal generator (10) and electric lead (11), passageways (41, 43) being provided in the connector body (25) to accept and guide the signal generator feedthrough pins (18, 19) and electrical lead (11) into contact with the terminal (40). The terminal (40) is provided with intersecting bores (51, 52) such that the respective feedthrough pins (18, 19) and electrical lead (11) form electrical contact within or by means of the terminal. The pins (18, 19) may be welded or press fit to the terminal (40) and the lead (11) may be attached to the terminal by means of a set screw (47). The preformed connector may be mechanically fastened or fastened with an adhesive to the implantable stimulator.

- 1 -

BODY IMPLANTABLE STIMULATOR AND CONNECTOR THEREFOR

This invention relates to a body implantable stimulator comprising a signal generator and a lead connector secured thereto. Such body implantable stimulators are known to the prior art, the most common being the cardiac or heart pacemaker. Typically, such stimulators are formed of a separable electrical lead and a signal generator with provision being made to electrically and mechanically connect the lead and generator to complete the stimulator unit.

Many prior art signal generators have been formed following assembly by casting the components, including mechanical and electrical connections for the lead, in a matrix of encapsulating material, typically epoxy resin, which supports the components and shields them from the body environment.

In the body environment, it is generally recognized that an enclosed and hermetically sealed signal generator is more reliable as a result of the known and controlled environment provided by the hermetic seal. For this reason, many recent signal generator designs include a rigid enclosure formed of a plurality of preformed members which are typically welded together to complete the enclosure. The connection between the generator and the electrical lead, when it is desired that these members be separable, occurs outside of such an enclosure. It is common to cast a connector from epoxy. However, it would be beneficial to eliminate the epoxy encapsulation process. Thus a preformed connector, which may be reliably secured to a preformed signal generator enclosure housing the generator components, would greatly facilitate assembly of the stimulator. The amount of handling would be reduced with the remaining handling being easier to perform than an epoxy casting process. One type of preformed connector assembly is disclosed in United States Patent Application Serial Number 793,642.

The above referenced application provides a preformed connector assembly thereby eliminating the necessity of forming that assembly in place, as by an epoxy casting process, for example. Additionally, in that assembly, the electrical connection between the connector assembly terminal and the signal generator requires manipulation of a wire to position it and a weld, or other similar process, to secure it in position. Thus, while the connector assembly of the above referenced application greatly reduces the handling necessary to form and complete a connector assembly on a signal generator unit, considerably handling remains necessary.

These shortcomings of the prior art are overcome by the invention as claimed. In one aspect, the connector is preformed and mechanical fastener means are provided for securing the connector to the generator. Thus, it eliminates the use of epoxy or other similar substances to encapsulate the terminal containing connector after attachment of the connector to the generator. This approach allows quality assurance of each preformed connector prior to assembly or attachment to the generator. The amount of handling necessary to assemble the stimulator and establish the proper electrical

- 3 -

connections is reduced.

In a preferred embodiment, the preformed connector is provided with first means, e.g. passageways which accept and guide the signal generator output connections (usually a feedthrough pin or pins) and preferably the electrical lead into electrical contact with a terminal in the connector. One or more terminals are provided with intersecting bores such that the feedthrough pin and electrical lead contact within the terminal or by means of it. Means are provided for securing the lead within the terminal. For example, this may be accomplished via a set screw which engages the lead to urge it against the terminal. The feedthrough pin may be welded or otherwise secured to the terminal as by a press fit for example.

In another aspect of the invention the connector comprises a preformed body formed of a moulded biocompatible material and a terminal enclosed in said body and adapted to receive and connect the output means of the generator and the lead wire, and including means attaching the connector to the generator.

A specific embodiment of the invention is now described by way of example only with reference to the drawings in which:-

Figure 1 is an exploded view of an embodiment of the present invention,

Figure 2 is a partial cutaway of the embodiment of Figure 1, as assembled,

- 4 -

Figure 3 is a cross section taken along the line 3-3 in Figure 2, and

Figure 4 is a cross section taken along the line 4-4 in Figure 3.

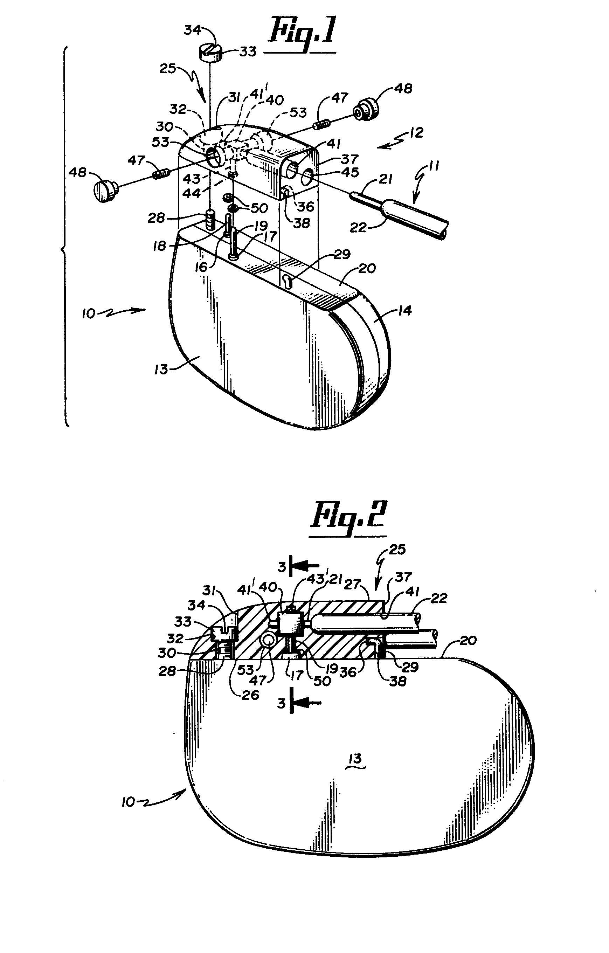

Referring now to Figure 1, there is illustrated an exploded view of a preferred embodiment of the electrical connector of the present invention. Figure 1 also shows generally an implantable signal generator 10, electrical lead 11, and the preformed connector 12 of the invention which, when assembled with the other parts, constitutes a heart pacemaker. Signal generator 10 includes all the necessary signal generating components, in known manner, the feedthroughs having electrical connections or feedthrough pins 18 and 19. Feedthroughs 16 and 17 extend from a surface 20 which is adapted to receive preformed connector 12 in a manner to be described more fully below. Electrical lead 11 is of the type having a pin connection 21 and insulating body 22 which surrounds and protects an electrical conductor (not shown). In use lead 11 extends from generator 10 to contact the heart muscle of the user to deliver pulses to the heart.

Connector 12 includes a body portion 25 which may be formed in any known manner, as by molding, for example.

- 5 -

Preferably, body 25 is of a clear material so as to allow visual verification of the electrical connections. Body 25 may be formed of many known materials including, polysulfone as sold under the tradename UDEL by Union Carbide, polyurethane as sold under the trade name PELLETHANE by Upjohn, polymethylpentene as sold under the tradename TPX by Mitsui and Company, polyvinylidene fluoride as sold under the tradename KYNAR, and ethylenechlorotrifluoroethylene as sold under the tradename HALAR by the Allied Chemical Corporation.

The undersurface 26 of body 25 is adapted to rest on surface 20 of signal generator 10 while the outer surface 27 is configured so as to extend the general outer configuration of signal generator 10 when surfaces 20 and 26 are mated. Many types of mechanical connections or adhesives may be used for this purpose. A typical mechanical arrangement is described herein for illustration although many other arrangements will be found to be satisfactory.

Extending from surface 20 is a threaded stud 28 and hook member 29. An aperture 30 extends from the under surface 26 of body 25 and joins a second aperture 31 extending from surface 27. Aperture 30 is large enough to accommodate threaded stud 28 while aperture 31 is large enough to accommodate a threaded nut 33. The junction of the apertures 30 and 31 forms a shoulder 32 on which nut 33 rests. Nut 33 is provided with a slot 34 so that it may be tightened on threaded stud 28 in the known manner. Of course, other tools may be employed requiring a different configuration in the recess shown as slot 34. For example, a hexagonal recess may be employed in conjunction with a tool of hexagonal cross section.

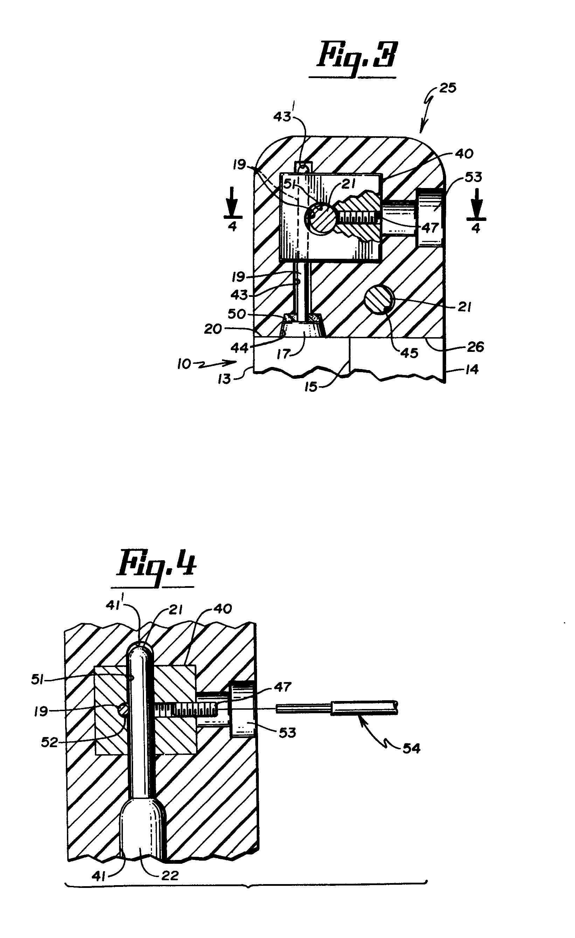

A second aperture 36 extends into body 25 from its face 37. Aperture 26 is adapted to accept the hook portion of hook 29 while the recess 38 on the face 37 is adapted to accept the lower portion of hook 29. On assembly, hook portion of hook 29 is inserted into recess 36 to engage its side wall and threaded stud 28 is inserted into aperture 30. Nut 33 then engages the threads on stud 28 and is tightened against the shoulder 32 to secure body 25 to the platform 20 and signal generator 10. This assembly is illustrated in Figure 2.

Contained within body 25 are conductive terminals 40, one terminal for each lead 11. The illustrated embodiment is intended for bi-polar stimulation. However, for the purposes of clarity, only one lead 11 and one terminal 40 are shown in Figure 1. A portion of a second lead 11 can be seen in Figure 2. An aperture 41 extends from face 37 of body 25 to terminal 40 with an extension 41 extending from terminal 40. Aperture 41 accepts lead 11 and guides pin 21 into electrical contact with terminal 40. Similarly, an aperture 43 extends from the undersurface 26 of body 25 to terminal 40 for the purpose of accepting and guiding feedthrough pin 19 into electrical contact with terminal 40. Aperture 43 includes an enlarged portion 44 which accommodates feedthrough 17. Similar apertures and terminals are provided for feedthrough 16 and feedthrough pin 18.

For example, an aperture 45 extends from face 37 to a terminal to accommodate a second lead. Aperture 53 in surface 27 allows access to set screws 47 carried by terminals 40 to lock pin 21 of lead 11 in position. Grommets 48 may be employed to seal the set screw apertures 53 while allowing access to the set screws, in known manner. Resilient washers 50 are provided which include a central aperture which accepts feedthrough pins 18 and 19 to rest atop feedthroughs 16 and 17. When the undersurface 26 of body 25 and surface 20 of generator 10 are mated, the shoulder formed between apertures 43 and 44 compresses the washers 50 against the top of feedthroughs 16 and 17 to seal pins 18 and 19 from the body environment.

Referring now to Figure 3, there is illustrated a cross section of body 25 taken along line 3-3 in Figure 2. As illustrated in Figure 3, terminal 40 includes a bore 51 which is adapted to accept pin 21 of lead 11. The aperture 41 of body 25 accepts lead 11 and guides pin 21 to and through bore 51. If body 25 is made of a transparent material, as preferred, the extension of pin 21 through bore 51 and into extension 41 provides visual assurance of proper placement of pin 21 relative to terminal 40. A second bore 52 in terminal 40 (see Figure 4) receives pin 19 of feedthrough 17, aperture 43 accepting pin 19 and guiding it to bore 52. An extension 43 of aperture 43 may be provided to allow visual verification of proper positioning of pin 19 relative to the terminal 40. In the illustration of Figure 3, lead 11 is not positioned within aperture 41 or bore 51 so as to illustrate the intersection of the bores 51 and 52 within terminal 40, pin 19 being visible through bore 51. As described above, an aperture 53 is provided for access to set screw 47. Set screw may be provided on its end with a hexagonal recess for cooperation with a tool 54 (see Figure 4) having a similar cross section at its terminus, in known manner.

Figure 4 is a cross section taken along line 4-4 in Figure 3 and further illustrates the intersection of bores 51 and 52, the intersection preferably being in line with the set screw. That is, as set screw 47 is tightened against pin 21, pin 21 is urged against feedthrough 19 thereby securing both pin 21 and feedthrough 19 in place within terminal 40. This further assures an electrical communication between feedthrough 19 and pin 21. However, other configurations may be employed so long as the pin 21 and feedthrough 19 are in contact with each other or with conductive terminal 40.

Obviously, many modifications and variations of the present invention are possible in light of the above teachings. As pointed out above, other securement systems may also be employed consistent with the present invention. Also, aperture 43 need not guide pin 19 through a bore in terminal 40, but, instead, need only guide it into contact with that terminal 40. If pin 19 is guided to a location adjacent to terminal 40, and the material for which the body 25 is made sufficiently transparent, pin 19 and terminal 40 may be welded to each other through the body material by known techniques. Further, pin 19 may be welded within the bore 52 of Figure 4 through the aperture engaged by the set screw 47 with the set screw 47 removed. Modification to accommodate unipolar stimulation is within the skill of one ordinarily skilled in the art. Accordingly, within the scope of the appended claims, the invention may be practised other than as specifically described.

CLAIMS

1.    A body implantable stimulator comprising a signal generator (10) and a lead connector (12) secured thereto, characterised in that said connector (12) is preformed and in that mechanical fastener means (28,29, 33) are provided for securing said connector (12) to said generator (10).

2.    A body implantable stimulator according to claim 1 in which said mechanical fastener means (28, 29, 33) engage said connector (12) at at least two spaced locations.

3.    A body implantable stimulator according to claim 1 or 2 in which said connector (12) is formed with a plurality of apertures (31, 36) and said mechanical fastener means (28, 29) extend from said signal generator (10) into said apertures (31, 36).

4.    A body implantable stimulator according to claim 4 including a member (34) threadedly engaging at least one of said fastener means (28).

5.    A body implantable stimulator according to claim 3 or 4 in which one of said mechanical fastener means comprises a hook (29) engaging the sidewall of one of said apertures (36).

6.    A body implantable stimulator according to any of the preceding claims in which the signal generator (10) includes output means (18, 19) extending therefrom and the connector (12) includes a plurality of terminals (40) for interconnecting said output means (18, 19) with a respective lead contact (21).

7.    A body implantable stimulator according to claim 6 in which said connector (12) includes first and second bores (41, 43) for accepting and guiding said output means (18, 19) and said contacts (21) into contact with said terminals (40).

8.    A body implantable stimulator according to claim 7

- 11 -

wherein said terminals (40) comprise first and second bores (51, 52) said first-mentioned bores (41, 43) accepting and guiding said output means (18, 19) and contacts (21) into respective terminal bores (51, 52).

9. A body implantable stimulator according to claim 8 in which said terminals (40) further comprise means (47) for locking said output means (18, 19) and contacts (21) in respective terminal bores (51, 52).

10. A body implantable stimulator according to claim 9 in which said locking means (47) engages a respective contact (21) and urges it against a respective output means (18, 19).

11. A body implantable stimulator according to claim 9 or 10 wherein said locking means comprises a set screw (47).

12. A body implantable stimulator according to claim 9 in which said locking means comprises a weld securing said output means (18, 19) in said terminal bores (51, 52).

13. A body implantable stimulator comprising a signal generator (10) having a contact (21), and a connector (12) including a terminal (40) carried by said signal generator (10) for electrically interconnecting said output means (18, 19) with said contact (21), characterised in that said connector (12) is preformed and includes first means (43) for accepting and guiding said output means (18, 19) into contact with said terminal (40).

14. A body implantable stimulator according to claim 13 in which said terminal (40) includes a bore (52) said first means (43) guiding said output means (18, 19) into said bore (52).

15. A body implantable stimulator according to claim 13 or 14 in which said connector (12) includes second means (41) for accepting said lead (11) and guiding said contact (21) into contact with said terminal (40).

16. A body implantable stimulator according to claim 15 in which said terminal (40) includes first and second bores (51, 52) and said first and second means (43, 41) receive and guide respectively said output means (18, 19) and said lead contact (21) into said first and second bores (51, 52).

17. A body implantable stimulator according to claim 16 in which said terminal (40) further comprises means (47) for locking said output means (18, 19) and lead contact (21) in said terminal bore.

18. A body implantable stimulator according to claim 17 in which said locking means (47) engages said contact (21) and urges it against said output means (18, 19).

19. A body implantable stimulator according to claim 17 or 18 in which said locking means (47) comprises a set screw.

20. A body implantable stimulator according to claim 17 in which said locking means comprises a weld securing said output means (18, 19) in said terminal bore (51, 52).

21. A body implantable stimulator according to any of claims 16 to 20 in which said first and second bores (51, 52) intersect within said terminal (40).

22. A body implantable stimulator comprising a signal generator (10) having output means (18, 19), a lead wire (11) and a pre-formed electrical connector (12) for electrically interconnecting said output means (18, 19) with said lead wire, characterised in that the connector (12) comprises a pre-formed body (25) formed of a moulded biocompatible material and a terminal (40) enclosed in said body (25) and adapted to receive and connect the output means (18, 19) and lead wire (11), and including means (28, 29, 33) attaching the connector (12) to the signal generator (10).

23. A body implantable stimulator according to claim 22 in

- 13 -

which the connector body (25) is substantially transparent.

24. A body implantable stimulator according to claim 22 or 23 in which the connector body (25) is formed with apertures (41, 43, 45) for receiving the lead wire (11) and output means (18, 19).

25. An electrical connector for interconnecting at least one output means (18, 19) with at least one respective lead wire (11), characterised in that the connector comprises a body (25) formed of a moulded biocompatible material and including a terminal (40) enclosed in said body (25) and adapted to receive and connect the output means (18, 19) and lead wire (11).

26. An electrical connector according to claim 25 in which the moulded material is substantially transparent.

27. An electrical connector according to claim 25 or 26 in which the moulded material is polysulfone, polyurethane, polymethylpentene, polyvinylidene fluoride, or ethylenechlorotrifluoroethylene.

28. An electrical conductor according to claim 25, 26 or 27 in which the body (25) comprises means (41, 43) for accepting and guiding the output means (18, 19) and lead wire (11) into contact with the terminal (40).

29. An electrical conductor according to claim 28 in which the terminal (40) includes first and second intersecting bores (51,52) and said accepting and guiding means (41, 43) serves to guide the output means (18, 19) and lead wire (11) into respective said bores.

30. An electrical connector according to claim 29 in which the terminal further includes a set screw (47) for locking the lead wire (11) in its bore (51).

0006281

*Fig.1*

*Fig.2*

*Fig.3*

*Fig.4*

| DOCUMENTS CONSIDERED TO BE RELEVANT | | | CLASSIFICATION OF THE APPLICATION (Int. Cl3) |
|---|---|---|---|
| Category | Citation of document with indication, where appropriate, of relevant passages | Relevant to claim | |
| | US - A - 3 683 933 (MANSFIELD) <br> * column 2, lines 47 to 62; figure 4 * <br> --- | 1,4,6, 7,13, 22,25, 28 | A 61 N 1/36 |
| | US - A - 4 072 387 (SOCHOR) <br> * column 4, lines 24 to 54; figure 5 * <br> --- | 5 | |
| | US - A - 3 544 955 (RUIZ) <br> * column 2, lines 1 to 64; figures 1,5 * <br> --- | 8-11, 14-19 21,29 30 | **TECHNICAL FIELDS SEARCHED (Int.Cl.²)** <br><br> A 61 N 1/36 <br> H 01 R 9/26 <br> H 01 R 9/24 <br> H 01 R 9/22 |
| | US - A - 4 072 154 (ANDERSON) <br> * column 4, line 64 to column 5, line 2; figure 3 * <br> --- | 10,11 13,15 17-19 22,23 25,26 | H 01 R 9/16 <br> H 01 R 4/28 <br> H 01 R 4/30 <br> H 01 R 13/52 <br> H 01 R 13/639 <br> H 01 R 4/36 |
| | US - A - 4 041 956 (PURDY) <br> * column 1, line 61 to column 2, line 3 * <br> --- | 27 | |
| A | FR - A - 2 268 510 (MEDTRONIC) <br> * page 4, lines 5 to 13; figure 1* <br> --- | 1 | **CATEGORY OF CITED DOCUMENTS** <br><br> X: particularly relevant <br> A: technological background <br> O: non-written disclosure <br> P: intermediate document |
| A | CH - A - 327 468 (WEBER) <br> * page 2, lines 62 to 72; figure 6 * <br> --- | 4,5 | T: theory or principle underlying the invention <br> E: conflicting application <br> D: document cited in the application <br> L: citation for other reasons |
| | | ./. | &: member of the same patent family, corresponding document |

The present search report has been drawn up for all claims

| Place of search | Date of completion of the search | Examiner |
|---|---|---|
| The Hague | 13-07-1979 | BIJN |

EPO Form 1503.1  06.78

# EUROPEAN SEARCH REPORT

**European Patent Office**

| DOCUMENTS CONSIDERED TO BE RELEVANT | | | CLASSIFICATION OF THE APPLICATION (Int. Cl.²) |
|---|---|---|---|
| Category | Citation of document with indication, where appropriate, of relevant passages | Relevant to claim | |
| E,P | <u>EP - A - 0 000 280</u> (TUNSTALL) <br><br> * page 8, lines 18 to 27; figure 5 * <br><br> ---------- | 1 | |
| | | | **TECHNICAL FIELDS SEARCHED (Int. Cl.²)** |

EPO Form 1503.2  06.78